# EUROPEAN PATENT SPECIFICATION

(11) **EP 3 551 266 B1**
(45) Date of publication and mention of the grant of the patent: **20.10.2021**
(21) Application number: 17808912.4
(22) Date of filing: 05.12.2017
(51) Int. Cl.: A61M 16/00, A61M 16/06

(54) **CUSHION MEMBER, PRESSURE SUPPORT SYSTEM INCLUDING SAME, AND METHOD OF MANUFACTURING SAME**
KISSENELEMENT, DRUCKUNTERSTÜTZUNGSSYSTEM DAMIT UND VERFAHREN ZUR HERSTELLUNG DAVON
ÉLÉMENT DE COUSSIN, SYSTÈME DE SUPPORT DE PRESSION LE COMPRENANT ET PROCÉDÉ POUR LE FABRIQUER

(30) Priority: 08.12.2016 US 201662431522 P
(43) Date of publication of application: 16.10.2019
(73) Proprietor: Koninklijke Philips N.V., 5656 AG Eindhoven (NL)
(72) Inventor: BAIKO, Robert William, 5656 AE Eindhoven (NL)
(74) Representative: Roche, Denis
(86) International application number: PCT/EP2017/081424
(87) International publication number: WO 2018/104253

(56) References cited:
- EP-A1- 2 705 870
- WO-A1-2010/016774
- WO-A1-2013/186650

## Description

### BACKGROUND OF THE INVENTION

### 1. Field of the Invention

The present invention relates to cushion members such as for example, cushion members for patient interface devices. The present invention also relates to pressure support systems including cushion members. The present invention also relates to methods of manufacturing cushion members.

### 2. Description of the Related Art

Many individuals suffer from disordered breathing during sleep. Sleep apnea is a common example of such sleep disordered breathing suffered by millions of people throughout the world. One type of sleep apnea is obstructive sleep apnea (OSA), which is a condition in which sleep is repeatedly interrupted by an inability to breathe due to an obstruction of the airway; typically the upper airway or pharyngeal area. Obstruction of the airway is generally believed to be due, at least in part, to a general relaxation of the muscles which stabilize the upper airway segment, thereby allowing the tissues to collapse the airway. Another type of sleep apnea syndrome is a central apnea, which is a cessation of respiration due to the absence of respiratory signals from the brain's respiratory center. An apnea condition, whether obstructive, central, or mixed, which is a combination of obstructive and central, is defined as the complete or near cessation of breathing, for example a 90% or greater reduction in peak respiratory airflow.

Those afflicted with sleep apnea experience sleep fragmentation and complete or nearly complete cessation of ventilation intermittently during sleep with potentially severe degrees of oxyhemoglobin desaturation. These symptoms may be translated clinically into extreme daytime sleepiness, cardiac arrhythmias, pulmonary-artery hypertension, congestive heart failure and/or cognitive dysfunction. Other consequences of sleep apnea include right ventricular dysfunction, carbon dioxide retention during wakefulness, as well as during sleep, and continuous reduced arterial oxygen tension. Sleep apnea sufferers may be at risk for excessive mortality from these factors as well as by an elevated risk for accidents while driving and/or operating potentially dangerous equipment.

Even if a patient does not suffer from a complete or nearly complete obstruction of the airway, it is also known that adverse effects, such as arousals from sleep, can occur where there is only a partial obstruction of the airway. Partial obstruction of the airway typically results in shallow breathing referred to as a hypopnea. A hypopnea is typically defined as a 50% or greater reduction in the peak respiratory airflow. Other types of sleep disordered breathing include, without limitation, upper airway resistance syndrome (UARS) and vibration of the airway, such as vibration of the pharyngeal wall, commonly referred to as snoring.

It is well known to treat sleep disordered breathing by applying a continuous positive air pressure (CPAP) to the patient's airway. This positive pressure effectively "splints" the airway, thereby maintaining an open passage to the lungs. It is also known to provide a positive pressure therapy in which the pressure of gas delivered to the patient varies with the patient's breathing cycle, or varies with the patient's breathing effort, to increase the comfort to the patient. This pressure support technique is referred to as bi-level pressure support, in which the inspiratory positive airway pressure (IPAP) delivered to the patient is higher than the expiratory positive airway pressure (EPAP). It is further known to provide a positive pressure therapy in which the pressure is automatically adjusted based on the detected conditions of the patient, such as whether the patient is experiencing an apnea and/or hypopnea. This pressure support technique is referred to as an auto-titration type of pressure support, because the pressure support device seeks to provide a pressure to the patient that is only as high as necessary to treat the disordered breathing.

Pressure support therapies as just described involve the placement of a patient interface device including a mask component having a soft, flexible sealing cushion on the face of the patient. The mask component may be, without limitation, a nasal mask that covers the patient's nose, a nasal/oral mask that covers the patient's nose and mouth, or a full face mask that covers the patient's face. Such patient interface devices may also employ other patient contacting components, such as forehead supports, cheek pads and chin pads. The patient interface device is typically secured to the patient's head by a headgear component. The patient interface device is connected to a gas delivery tube or conduit and interfaces the pressure support device with the airway of the patient, so that a flow of breathing gas can be delivered from the pressure/flow generating device to the airway of the patient.

One drawback of known patient interface devices is that during therapy, leaks often form between the sealing portion of the cushion member and the patient's face. Buckling of the sealing portions is the leading cause of leak experienced by a CPAP mask. When a buckle forms, a leak path is created that propagates to an exterior environment. Leaks cause rushes of gas flow against the patient's skin, which can prevent a patient from remaining asleep, disturb a bed partner, and also compromise the quality of therapy being delivered to the patient.

The patent document WO2010/016774A1 discloses a respiratory mask sealing interface.

### SUMMARY OF THE INVENTION

The invention is defined by the appended claims.

Accordingly, it is an object of the present invention to provide an improved cushion member, pressure support system including the same, and a method of manufacturing the same.

As one aspect of the disclosed concept, a cushion member is provided for use in a pressure support system for delivering a flow of breathing gas to an airway of a patient. The cushion member includes a body portion having a first end and a second end located opposite the first end, the body portion defining a passage therethrough; and a sealing portion structured to sealingly engage about the airway of the patient. The sealing portion extends radially inwardly from the first end to an edge portion. The sealing portion has a notched portion defined therein which extends outward from the edge portion.

As another aspect of the disclosed concept, a pressure support system including a gas flow generator, a conduit coupled to the gas flow generator, and the aforementioned cushion member is provided.

As another aspect of the disclosed concept, a method of manufacturing the aforementioned cushion member is provided.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a simplified, partially schematic, view of a pressure support system, in accordance with a non-limiting embodiment of the disclosed concept;
FIG. 2 is an elevation view of the user facing side of a cushion member for the pressure support system of FIG. 1;
FIG. 3 is an elevation view of a portion of the cushion member of FIG. 2, shown in a first configuration;
FIG. 4 is another elevation view of the portion of the cushion member of FIG. 2, shown in a second configuration;
FIG. 5 is a side elevation view of the portion of FIG. 4;
FIG. 6 is a side elevation view of a portion of a prior art cushion member; and
FIGS. 7-9 are elevation views of portions of other cushion members, in accordance with other non-limiting embodiments of the disclosed concept.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other.

As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

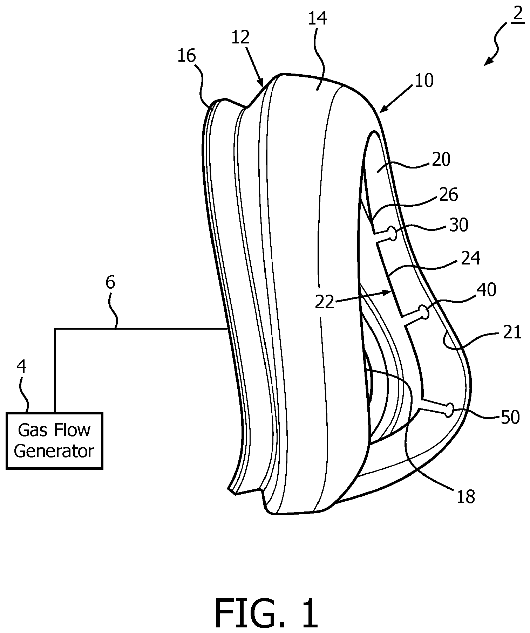

FIG. 1 is a simplified, partially schematic, view of a pressure support system 2, in accordance with one non-limiting embodiment of the disclosed concept. Pressure support system 2 includes, among other components, a gas flow generator 4 (shown in simplified form), a conduit (e.g., without limitation, hose 6, shown in simplified form), and a cushion member 10. Gas flow generator 4 is structured to generate a flow of breathing gas to be delivered to an airway of a patient. Hose 6 fluidly couples gas flow generator 4 to cushion member 10 via a suitable frame member (not shown) and/or a fluid coupling conduit (not shown) in order to allow pressure support therapy to be delivered to an airway of a patient.

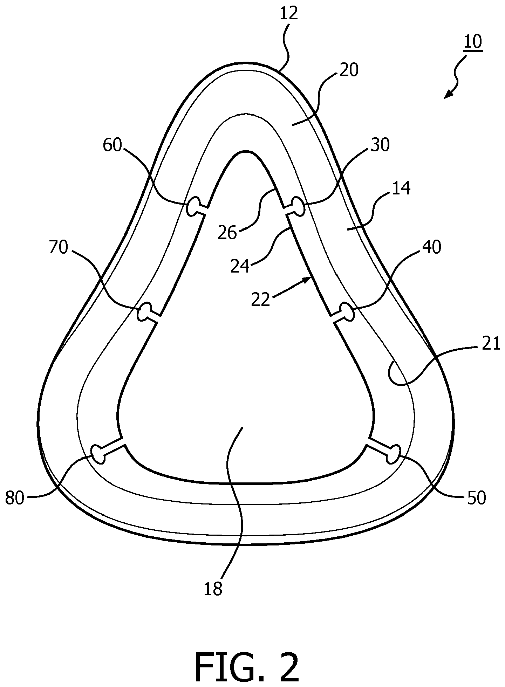

Cushion member 10 includes a body portion 12 having a first end 14 and a second end 16 located opposite first end 14. Second end 16 may be directly or indirectly coupled to hose 6. Body portion 12 defines a passage 18 therethrough which receives a flow of breathing gas from gas flow generator 4 from hose 6. Cushion member 10 further includes a sealing portion 20 extending radially inwardly from first end 14 into passage 18. In operation, sealing portion 20 is structured to sealingly engage about an airway of a patient. As shown, sealing portion 20 extends radially inwardly from first end 14 to an edge portion 22. Sealing portion 20 further has a number of novel notched portions 30, 40, 50, 60, 70 (FIG. 2), 80 (FIG. 2) defined therein which each extend outward from edge portion 22. Notched portions 30, 40, 50, 60, 70 (FIG. 2), 80 (FIG. 2) each define a respective void, or cutout region, in sealing portion 20.

For ease of illustration and economy of disclosure, only notched portion 30 will be described in detail herein, although it will be appreciated that notched portions 40, 50, 60, 70, 80 are structured and function substantially the same as notched portion 30. As shown in FIG. 1 and FIG. 2, edge portion 22 includes a first portion 24 and a second portion 26. Notched portion 30 extends outward from first portion 24 and second portion 26. Additionally, although the disclosed concept is being described herein in association with full face cushion member 10, it will be appreciated that any suitable alternative cushion member (e.g., without limitation, cradle cushion member, nasal cushion member, pillows style cushion member, not shown) may have notched portions similar to notched portions 30, 40, 50, 60, 70,80, without departing from the scope of the disclosed concept.

It will be appreciated that sealing portion 20 curls generally away from first end 14 toward second end 16. Accordingly, sealing portion 20 further includes an annular-shaped sealing region 21 (i.e., a generally central region of curved sealing portion 20 located opposite and distal second end 16 that engages the face of the patient to provide a seal therewith) located between first end 14 and edge portion 22. As shown, notched portion 30 is located between sealing region 21 and edge portion 22. Notched portion 30 also extends from first portion 24 and second portion 26 outwardly toward first end 14.

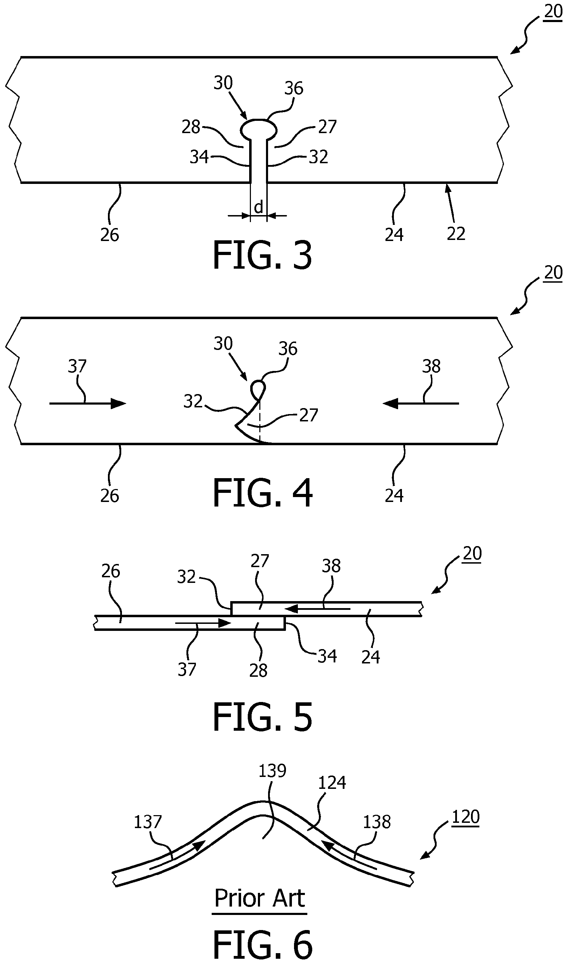

As shown in FIG. 3, notched portion 30 includes a first edge portion 32, a second edge portion 34 spaced a distance d from first edge portion 32, and a curved edge portion (e.g., without limitation, partially circular-shaped edge portion 36) connecting first edge portion 32 to second edge portion 34. In the exemplary embodiment, edge portions 32 ,34 are linear, parallel to one another, and perpendicular to respective portions 24, 26. However, it is to be appreciated that edge portions 32, 34 may be of other shape (i.e., non-linear) and of other orientations, without departing from the scope of the disclosed concept. That is, suitable alternative edge portions (not shown) may be curved and/or may be oriented at acute or obtuse angles with respect to inner edge portions of a sealing portion. Additionally, in one example embodiment, the ratio of d to the length of edge portions 32, 34 is between 0.005 and 0.5. Furthermore, sealing portion 20 further includes a number of side portions (two side portions 27,28 are shown in FIG. 3). First side portion 27 extends from and is adjacent first portion 24 and first edge portion 32. Second side portion 28 extends from and is adjacent second portion 26 and second edge portion 34.

Sealing portion 20 is structured to move from a first configuration (FIG. 3) to a second configuration (FIGS. 4 and 5). Sealing portion 20 moves from the first configuration (FIG. 3) to the second configuration (FIGS. 4 and 5) responsive to, for example and without limitation, circumferential compression that occurs when cushion member 10 engages the face of a patient. See, for example, arrows 37,38 in FIG. 4, which represent compressive forces along the circumference of sealing portion 20. When sealing portion 20 is in the first configuration (FIG. 3), edge portion 32 is spaced from edge portion 34. When sealing portion 20 moves from the first configuration (FIG. 3) to the second configuration (FIGS. 4 and 5), first side portion 27 advantageously overlaps second side portion 28 such that in the second configuration (FIGS. 4 and 5), first side portion 27 is sealingly abuts second side portion 28. See, for example, the side elevation view of FIG. 5 in which first side portion 27 sealingly abuts second side portion 28. As such, in the second configuration (FIGS. 4 and 5), first side portion 27 sealingly engages second side portion 28 such that sealing portion 20 is substantially impervious to the flow of gas between first side portion 27 and second side portion 28. Additionally, in the example of FIGS. 3 - 5, when sealing portion 20 moves from the first configuration (FIG. 3) to the second configuration (FIGS. 4 and 5), first edge portion 32 pivots about partially circular-shaped edge portion 36. Accordingly, the curved geometry of edge portion 36 advantageously allows for the beneficial overlapping of side portions 27,28. In the exemplary embodiment, edge portion 36 is partially circular-shaped. However, it will be appreciated that a suitable alternative edge portion may have other geometries (e.g., without limitation, partially square-shaped, half-moon-shaped, not shown), without departing from the scope of the disclosed concept.

FIG. 6 shows a side elevation view of a portion of a prior art sealing portion 120. It will be appreciated that sealing portion 120 does not have any notched portions, but rather has one single continuous edge portion 124. Arrows 137, 138 represent compressive forces along the circumference of sealing portion 120 that may occur when a cushion member including sealing portion 120 engages the face of a patient. Compressive forces 137, 138 in sealing portion 120 cause an undesirable buckle to form such that a passageway 139 is provided through which breathing gas can escape, or leak out, from an interior of a respective cushion member to an exterior thereof.

This is distinct from the novel cushion member 10. More specifically, as shown in FIG. 5, compressive forces 37, 38 do not cause an undesirable passageway to form for breathing gas to escape, but rather cause side portions 27, 28 to overlap such that an effective seal can be maintained. Furthermore, because notched portions 30, 40, 50, 60, 70, 80 are located between sealing region 21 and edge portion 22, all overlapping between side portions 27, 28 is advantageously contained between the primary engaging portion (i.e., sealing region 21) of sealing portion 20 and interior edge portion 22 thereof. In this manner, the possibility of leaks forming between sealing portion 20 and the face of a patient when pressure support therapy is being delivered is significantly minimized. That is, the possibility of a buckle propagating to the sealing surface is substantially reduced. As such, the quality of pressure support therapy being delivered to the patient is advantageously improved.

A method of manufacturing cushion member 10 includes the steps of providing body portion 12, providing sealing portion 20 extending radially inwardly from first end 14 to edge portion 22, and providing a notched portion 30 extending outward from edge portion 22. The providing a notched portion 30 step may further include removing a predetermined quantity of material from sealing portion 20. For example and without limitation, removing the predetermined quantity of material from sealing portion 20 may include diecutting sealing portion 20 to remove the material. The providing a notched portion 30 step may alternatively further include molding sealing portion 20 with notched portions 30.

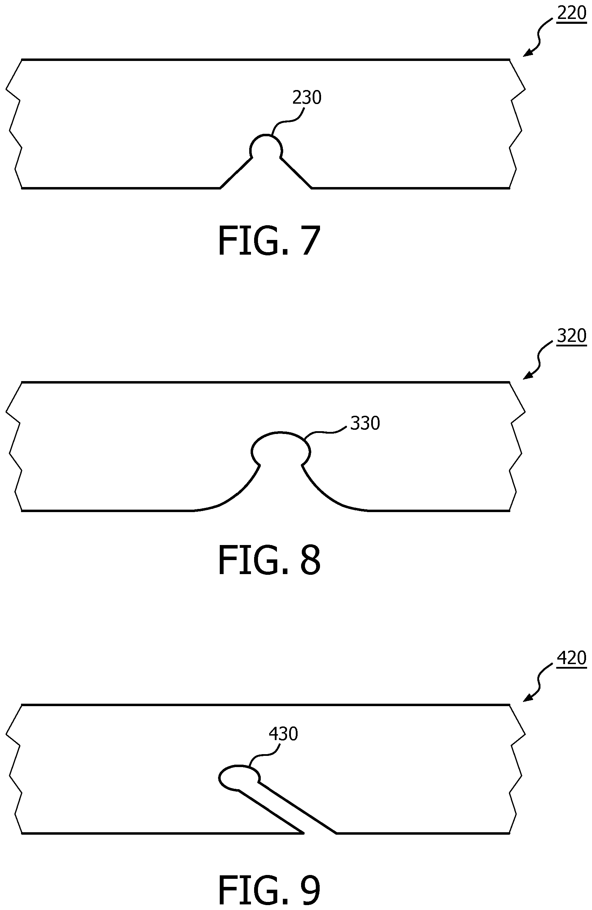

FIGS. 7-9 show portions of other sealing portions 220, 320, 420, in accordance with other non-limiting embodiments of the disclosed concept. As shown, sealing portions 220, 320, 420 include notched portions 230, 330, 430 that have edge portions that are at acute angles with respect to each other, curved, and at acute angles with respect to inner edge portions, respectively. As such, the disclosed concept provides for notched portions 30, 40, 50, 60, 70, 80, 230, 330, 430 having many suitable geometries. Additionally, it will be appreciated that the edge portions of notched portions 230,330 need not overlap one another, but rather can move toward, but not contact one another when moving from a first configuration to a second configuration (not shown), within the scope of the disclosed concept.

Accordingly, it will be appreciated that the disclosed concept provides for an improved (e.g., without limitation, substantially reduced likelihood for leaks) cushion member 10, pressure support system 2 including the same, and method of manufacturing the same, in which a number of notched portions 30, 40, 50, 60, 70, 80, 230, 330, 430 are provided on a sealing portion 20 of cushion member 10 to substantially reduce the propagation of buckles from an interior of cushion member 10 to an exterior thereof.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims.

## Claims

1. A cushion member (10) for use in a pressure support system (2) for delivering a flow of breathing gas to an airway of a patient, the cushion member comprising:
a body portion (12) comprising a first end (14) and a second end (16) disposed opposite the first end, the body portion defining a passage (18) therethrough; and
a sealing portion (20) structured to sealingly engage about the airway of the patient, the sealing portion extending radially inwardly from the first end to an edge portion (22), the sealing portion having a notched portion (30) defined therein which extends outward from the edge portion, wherein the sealing portion further comprises an annular-shaped sealing region (21) disposed between the first end and the edge portion, wherein the sealing region is a central region of the sealing portion, and wherein the notched portion is disposed between the sealing region and the edge portion.

2. The cushion member according to claim 1, wherein the notched portion is defined in the sealing portion by a first edge portion (32), a second edge portion (34) spaced a distance (d) from the first edge portion, and a partially circular-shaped edge portion (36) connecting the first edge portion to the second edge portion.

3. The cushion member according to claim 2, wherein the first edge portion and the second edge portion are each linear.

4. The cushion member according to claim 3, wherein the first edge portion is parallel to the second edge portion.

5. The cushion member according to claim 2, wherein the sealing portion is structured to move from a first configuration wherein the first edge portion is spaced from the second edge portion to a second configuration wherein a portion (27) of the sealing portion adjacent the first edge portion overlaps another portion (28) of the sealing portion adjacent the second edge portion.

6. The cushion member according to claim 5, wherein, when the sealing portion is in the second configuration, the portion of the sealing portion adjacent the first edge portion sealingly engages the other portion of the sealing portion adjacent the second edge portion.

7. The cushion member according to claim 1, wherein the sealing portion curls away from the first end toward the second end.

8. The cushion member according to claim 1, wherein the sealing portion further comprises a number of other notched portions (40,50,60,70,80) formed therein.

9. A pressure support system (2) comprising:
a gas flow generator (4);
a conduit (6) coupled to the gas flow generator;
a cushion member (10) comprising:
a body portion (12) comprising a first end (14) and a second end (16) disposed opposite the first end, the body portion defining a passage (18) therethrough, the second end being coupled to the conduit, and
a sealing portion (20) structured to sealingly engage about the airway of the patient, the sealing portion extending radially inwardly from the first end to an edge portion (22), the sealing portion having a notched portion (30) defined therein which extends outward from the edge portion, wherein the sealing portion further comprises an annular-shaped sealing region (21) disposed between the first end and the edge portion;
wherein the sealing region is a central region of the sealing portion; and wherein the notched portion is disposed between the sealing region and the edge portion.

10. The pressure support system according to claim 9, wherein the notched portion is defined in the sealing portion by a first edge portion (32), a second edge portion (34) spaced a distance (d) from the first edge portion, and a partially circular-shaped edge portion (36) connecting the first edge portion to the second edge portion.

11. The pressure support system according to claim 10, wherein the first edge portion and the second edge portion are each linear; and wherein the first edge portion is parallel to the second edge portion.

12. A method of manufacturing a cushion member (10), the method comprising the steps of:
providing a body portion (12) comprising a first end (14) and a second end (16) disposed opposite the first end, the body portion defining a passage (18) therethrough;
providing a sealing portion (20) structured to sealingly engage about an airway of a patient, the sealing portion extending radially inwardly from the first end to an edge portion; and
providing a notched portion (30) in the sealing portion, the notched portion extending outward from the edge portion, wherein the sealing portion further comprises an annular-shaped sealing region (21) disposed between the first end and the edge portion; wherein the sealing region is a central region of the sealing portion; and wherein the notched portion is disposed between the sealing region and the edge portion.

13. The method according to claim 12, wherein providing the notched portion comprises removing a predetermined quantity of material from the sealing portion.

14. The method according to claim 13, wherein removing the predetermined quantity of material comprises diecutting the sealing portion to remove the material.

15. The method according to claim 12, wherein providing the notched portion comprises molding the sealing portion with the notched portion defined therein.

## Patentansprüche

1. Ein Polsterelement (10) für ein Atemwegsdruck-Unterstützungssystem (2) zum Zuführen eines Atemgasflusses in die Atemwege eines Patienten,
wobei das Polsterelement Folgendes umfasst:
ein Gehäuseteil (12) mit einem ersten Ende (14) und einem zweiten, dem ersten Ende gegenüberliegenden Ende (16), wobei das Gehäuseteil einen Durchlass (18) definiert; und
einen Dichtungsteil (20), der die Atemwege des Patienten abdichtend umschließt, wobei sich der Dichtungsteil vom ersten Ende radial nach innen hin zu einem Randabschnitt (22) erstreckt, wobei sich im Dichtungsteil ein gekerbter Abschnitt (30) befindet, der sich vom Randabschnitt nach außen erstreckt,
wobei der Dichtungsteil zudem einen ringförmigen Dichtungsbereich (21) aufweist, der sich zwischen dem ersten Ende und dem Randabschnitt befindet,
wobei es sich beim Dichtungsbereich um den zentralen Bereich des Dichtungsabschnitts handelt, und
wobei sich der gekerbte Abschnitt zwischen dem Dichtungsbereich und dem Randabschnitt befindet.

2. Das Polsterelement gemäß Anspruch 1, wobei der gekerbte Abschnitt des Dichtungsteils durch einen ersten Randabschnitt (32), einen zweiten Randabschnitt (34), der einen Abstand (d) zum ersten Randabschnitt aufweist, und einen teilweise kreisförmigen Randabschnitt (36) definiert wird, der den ersten Randabschnitt mit dem zweiten Randabschnitt verbindet.

3. Das Polsterelement gemäß Anspruch 2, wobei der erste Randabschnitt und der zweite Randabschnitt jeweils linear sind.

4. Das Polsterelement gemäß Anspruch 3, wobei der erste Randabschnitt parallel zum zweiten Randabschnitt verläuft.

5. Das Polsterelement gemäß Anspruch 2, wobei der Dichtungsteil sich von einer ersten Konfiguration, in der der erste Randabschnitt einen Abstand zum zweiten Randabschnitt aufweist, in eine zweite Konfiguration bewegen lässt, in der ein an den ersten Randabschnitt angrenzender Teil (27) des Dichtungsteils einen weiteren an den zweiten Randabschnitt angrenzenden Teil (28) des Dichtungsteils überlappt.

6. Das Polsterelement gemäß Anspruch 5, wobei, wenn sich der Dichtungsteil in der zweiten Konfiguration befindet, der an den ersten Randabschnitt angrenzende Teil des Dichtungsteils in den an den zweiten Randabschnitt angrenzenden anderen Teil des Dichtungsteils einrastet und diesen abdichtet.

7. Das Polsterelement gemäß Anspruch 1, wobei sich der Dichtungsteil am ersten Ende beginnend hin zum zweiten Ende wölbt.

8. Das Polsterelement gemäß Anspruch 1, wobei der Dichtungsteil zudem mehrere weitere gekerbte Abschnitten (40, 50, 60, 70, 80) umfasst.

9. Ein Druckunterstützungssystem (2), das Folgendes umfasst:
einen Gasströmungsgenerator (4);
eine mit dem Gasströmungsgenerator verbundene Leitung (6) ;
ein Polsterelement (10), das Folgendes umfasst:
ein Gehäuseteil (12) mit einem ersten Ende (14) und einem zweiten, dem ersten Ende gegenüberliegenden Ende (16), wobei das Gehäuseteil einen Durchlass (18) definiert; und
wobei das zweite Ende mit der Leitung verbunden ist, und
einen Dichtungsteil (20), der die Atemwege des Patienten abdichtend umschließt, wobei sich der Dichtungsteil vom ersten Ende radial nach innen hin zu einem Randabschnitt (22) erstreckt, wobei sich im Dichtungsteil ein gekerbter Abschnitt (30) befindet, der sich vom Randabschnitt nach außen erstreckt, wobei der Dichtungsteil zudem einen ringförmigen Dichtungsbereich (21) aufweist, der sich zwischen dem ersten Ende und dem Randabschnitt befindet;
wobei es sich beim Dichtungsbereich um den zentralen Bereich des Dichtungsabschnitts handelt; und
wobei sich der gekerbte Abschnitt zwischen dem Dichtungsbereich und dem Randabschnitt befindet.

10. Das Druckunterstützungssystem gemäß Anspruch 9, wobei der gekerbte Abschnitt des Dichtungsteils durch einen ersten Randabschnitt (32), einen zweiten Randabschnitt (34), der einen Abstand (d) zum ersten Randabschnitt aufweist, und einen teilweise kreisförmigen Randabschnitt (36) definiert wird, der den ersten Randabschnitt mit dem zweiten Randabschnitt verbindet.

11. Das Druckunterstützungssystem gemäß Anspruch 10, wobei der erste Randabschnitt und der zweite Randabschnitt jeweils linear sind, und
wobei der erste Randabschnitt parallel zum zweiten Randabschnitt verläuft.

12. Eine Methode zum Herstellen eines Polsterelements (10), wobei die Methode folgende Schritte umfasst:
Bereitstellen eines Gehäuseteils (12) mit einem ersten Ende (14) und einem zweiten, dem ersten Ende gegenüberliegenden Ende (16), wobei das Gehäuseteil einen Durchlass (18) definiert;
Bereitstellen eines Dichtungsteils (20), der die Atemwege des Patienten abdichtend umschließt, wobei sich der Dichtungsteil vom ersten Ende radial nach innen hin zu einem Randabschnitt erstreckt; und Bereitstellen eines gekerbten Abschnitts (30) im Dichtungsteil, wobei sich der gekerbte Abschnitt vom Randabschnitt nach außen erstreckt, wobei der Dichtungsteil zudem einen ringförmigen Dichtungsbereich (21) aufweist, der sich zwischen dem ersten Ende und dem Randabschnitt befindet;
wobei es sich beim Dichtungsbereich um den zentralen Bereich des Dichtungsabschnitts handelt; und
wobei sich der gekerbte Abschnitt zwischen dem Dichtungsbereich und dem Randabschnitt befindet.

13. Die Methode gemäß Anspruch 12, wobei das Bereitstellen des gekerbten Abschnitts das Entfernen einer vorab festgelegten Materialmenge vom Dichtungsteil umfasst.

14. Die Methode gemäß Anspruch 13, wobei das Entfernen der vorab festgelegten Materialmenge das Stanzen des Dichtungsteils umfasst, um das Material zu entfernen.

15. Die Methode gemäß Anspruch 12, wobei das Bereitstellen des gekerbten Abschnitts das Formen des Dichtungsteils mit dem darin befindlichen gekerbten Abschnitt umfasst.

## Revendications

1. Élément coussin (10) destiné à être utilisé dans un système de support de pression (2) pour délivrer un écoulement de gaz respiratoire à des voies aériennes d'un patient, l'élément coussin comprenant :
une partie corps (12) comprenant une première extrémité (14) et une seconde extrémité (16) disposée à l'opposé de la première extrémité, la partie corps définissant un passage (18) à travers celle-ci ; et
une partie d'étanchéité (20) conçue pour entrer en prise de manière étanche autour des voies respiratoires du patient, la partie d'étanchéité s'étendant radialement vers l'intérieur depuis la première extrémité jusqu'à une partie de bord (22), la partie d'étanchéité comportant une partie encochée (30) définie à l'intérieur, laquelle s'étend vers l'extérieur de la partie de bord, dans lequel la partie d'étanchéité comprend en outre une zone d'étanchéité (21) annulaire disposée entre la première extrémité et la partie de bord, dans lequel la zone d'étanchéité est une zone centrale de la partie d'étanchéité, et dans lequel la partie encochée est disposée entre la zone d'étanchéité et la partie de bord.

2. Élément coussin selon la revendication 1, dans lequel la partie encochée est définie dans la partie d'étanchéité par une première partie de bord (32), une deuxième partie de bord (34), espacée d'une distance (d) de la première partie de bord, et une partie de bord (36) partiellement circulaire reliant la première partie de bord à la deuxième partie de bord.

3. Élément coussin selon la revendication 2, dans lequel la première partie de bord et la deuxième partie de bord sont respectivement linéaires.

4. Élément coussin selon la revendication 3, dans lequel la première partie de bord est parallèle à la deuxième partie de bord.

5. Élément coussin selon la revendication 2, dans lequel la partie d'étanchéité est conçue pour se déplacer à partir d'une première configuration, dans lequel la première partie de bord est espacée de la deuxième partie de bord à une seconde configuration, dans lequel une partie (27) de la partie d'étanchéité adjacente à la première partie de bord chevauche une autre partie (28) de la partie d'étanchéité adjacente à la deuxième partie de bord.

6. Élément coussin selon la revendication 5, dans lequel, lorsque la partie d'étanchéité est dans la seconde configuration, la partie de la partie d'étanchéité adjacente à la première partie de bord entre en prise de manière étanche avec l'autre partie de la partie d'étanchéité adjacente à la deuxième partie de bord.

7. Élément coussin selon la revendication 1, dans lequel la partie d'étanchéité s'enroule de la première extrémité vers la seconde extrémité.

8. Élément coussin selon la revendication 1, dans lequel la partie d'étanchéité comprend en outre un nombre d'autres parties encochées (40, 50, 60, 70, 80) formées à l'intérieur de celle-ci.

9. Système de support de pression (2) comprenant :
un générateur d'écoulement de gaz (4) ;
un conduit (6) couplé au générateur d'écoulement de gaz ;
un élément coussin (10) comprenant :
une partie corps (12) comprenant une première extrémité (14) et une seconde extrémité (16), disposée à l'opposé de la première extrémité, la partie corps définissant un passage (18) à travers celle-ci, la seconde extrémité étant couplée au conduit, et
une partie d'étanchéité (20) conçue pour entrer en prise de manière étanche autour des voies respiratoires du patient, la partie d'étanchéité s'étendant radialement vers l'intérieur depuis la première extrémité jusqu'à une partie de bord (22), la partie d'étanchéité comportant une partie encochée (30) définie à l'intérieur de celle-ci, laquelle s'étend vers l'extérieur de la partie de bord, dans lequel la partie d'étanchéité comprend en outre une zone d'étanchéité (21) annulaire disposée entre la première extrémité et la partie de bord ;
dans lequel la zone d'étanchéité est une zone centrale de la partie d'étanchéité ; et
dans lequel la partie encochée est disposée entre la zone d'étanchéité et la partie de bord.

10. Système de support de pression selon la revendication 9, dans lequel la partie encochée est définie dans la partie d'étanchéité par une première partie de bord (32), une deuxième partie de bord (34), espacée d'une distance (d) à partir de la première partie de bord, et une partie de bord (36) partiellement circulaire reliant la première partie de bord à la deuxième partie de bord.

11. Système de support de pression selon la revendication 10, dans lequel la première partie de bord et la deuxième partie de bord sont respectivement linéaires ; et dans lequel la première partie de bord est parallèle à la deuxième partie de bord.

12. Procédé de fabrication d'un élément coussin (10), le procédé comprenant les étapes suivantes :
la fourniture d'une partie corps (12) comprenant une première extrémité (14) et une seconde extrémité (16), disposée à l'opposé de la première extrémité, la partie corps définissant un passage (18) à travers celle-ci ;
la fourniture d'une partie d'étanchéité (20) conçue pour entrer en prise de manière étanche autour des voies aériennes d'un patient, la partie d'étanchéité s'étendant radialement vers l'intérieur depuis la première extrémité jusqu'à une partie de bord ; et
la fourniture d'une partie encochée (30) dans la partie d'étanchéité, la partie encochée s'étendant vers l'extérieur à partir de la partie de bord, dans lequel la partie d'étanchéité comprend en outre une zone d'étanchéité (21) annulaire disposée entre la première extrémité et la partie de bord ;
dans lequel la zone d'étanchéité est une zone centrale de la partie d'étanchéité ; et
dans lequel la partie encochée est disposée entre la zone d'étanchéité et la partie de bord.

13. Procédé selon la revendication 12, dans lequel la fourniture de la partie encochée comprend l'enlèvement d'une quantité prédéterminée de matière de la partie d'étanchéité.

14. Procédé selon la revendication 13, dans lequel le retrait de la quantité prédéterminée de matière comprend le découpage de la partie d'étanchéité pour enlever la matière.

15. Procédé selon la revendication 12, dans lequel la fourniture de la partie encochée comprend le moulage de la partie d'étanchéité avec la partie encochée définie à l'intérieur de celle-ci.
